# EUROPÄISCHE PATENTSCHRIFT

(11) **EP 0 629 237 B1**
(45) Veröffentlichungstag und Bekanntmachung des Hinweises auf die Patenterteilung: **20.12.1995**
(21) Anmeldenummer: 93906477.0
(22) Anmeldetag: 02.03.1993
(51) Int. Cl.: C12M 3/06

(54) **VORRICHTUNG ZUR BEHANDLUNG VON ZELLKULTUREN**
DEVICE FOR CELL CULTURE TREATMENT
DISPOSITIF POUR LE TRAITEMENT DE CULTURES CELLULAIRES

(30) Priorität: 03.03.1992 DE 4206585
(43) Veröffentlichungstag der Anmeldung: 21.12.1994
(73) Patentinhaber: BADER, Augustinus, D-31275 Lehrte (DE)
(72) Erfinder: BADER, Augustinus, D-31275 Lehrte (DE)
(74) Vertreter: Lorenz, Werner, Dipl.-Ing.
(86) Internationale Anmeldenummer: EP9300468
(87) Internationale Veröffentlichungsnummer: WO9318133

(56) Entgegenhaltungen:
- EP-A- 0 180 165
- EP-A- 0 232 975
- EP-A- 0 363 262
- EP-A- 0 396 138
- EP-A- 0 416 586
- WO-A-89/12676
- WO-A-90/12604
- US-A- 4 201 845

## Beschreibung

Die Erfindung betrifft eine Vorrichtung zur Behandlung von Zellkulturen, insbesondere von Leberzellen (Hepatozyten), auf Zellkulturträgern, wobei wenigstens ein Teil der Oberflächen der Zellkulturträger gasdurchlässig ist.

In der Medizin und in der Pharmazie ist es oft erforderlich, Versuche mit Zellkulturen durchzuführen. Dies gilt z.B. zu deren Zucht, zu deren Beobachtung, deren Reaktion auf Fremd- und/oder Giftstoffe, zur Konservierung und dergleichen.

Darüber hinaus wird die Suche nach geeigneten Organersatzen immer wichtiger.

Eines der Hauptgebiete sind Versuche bezüglich Stoffwechselfunktionen, insbesondere der Leber.

Die Komplexität der vielfältigen hepatozellulären Stoffwechselfunktionen stellt jedoch hohe Anforderungen an einen künstlichen Organersatz für die Leber. Bei der künstlichen Niere stehen Filtrations- und Stoffaustauschaufgaben im Vordergrund, die durch ein Gerät im Sinne einer Dialyse erfüllt werden können. Ähnlich wird bei einem künstlichen Herzen vor allem die Pumpfunktion durch eine Maschine ersetzt. Die Leber hingegen besitzt eine Vielzahl von Einzelfunktionen, die sich grob in Kategorien wie Entgiftungsfunktion, Proteinsekretion, endokrine Aufgaben, Speicherfunktion, Phagozytose, Fett- und Kohlenhydratstoffwechselaufgaben untergliedern lassen.

Bei bekannten Kultursystemen verlieren die Leberzellen, nämlich die Hepatozyten, schon innerhalb der ersten Tage nach Isolation ihre Funktionsfähigkeit. So sind bereits nach 2 bis 3 Tagen, je nach untersuchter Funktion, nur noch ca. 80 % und nach 1 Woche nur noch minimale Restfunktionen erhalten. Danach kommt es zum Zelltod und zum Überwuchern mit fibroblastenartigen Zellen. Bisherige Experimente mit Leberzellkulturen mußten in einer Phase mit fortschreitendem Zellverfall durchgeführt werden.

Zum Erhalt der hepatozytären Funktion in Kultur wurde z.B. schon vorgeschlagen, die epitheliale Kokultur, die Hinzufügung von Dimethylsulfoxid (DMSO) zum Medium, oder die Verwendung einer komplexen Matrix (Matrigel) zu praktizieren. Ist das Ziel jedoch die Verwendung einer Leberzellkultur, die der "in vivo"-Situation möglichst nahe kommen soll, so entstehen durch diese herkömmlichen Kulturtechniken eine Reihe von Problemen. So ist DMSO eine chemische Substanz, die auch hepatotoxische Wirkung besitzt. Epitheliale Kokulturen sind transformierte Zellinien und haben onkogenen Charakter. Rückschlüsse auf das Verhalten natürlicher volldifferenzierter Zellen sind daher nicht zweifelsfrei möglich. Matrigel ist wiederum von Sarkomzellinien abgeleitet (Engelbrecht Holm Sarkom) und in seinen Komponenten nicht charakterisiert. Ein klinischer Einsatz onkogener Zellen oder deren nicht näher definierter Produkte ist daher nicht erstrebenswert.

Es wurde auch bereits ein System vorgeschlagen, wobei dieses im allgemeinen aus einem Hepatozytenmonolayer unter Anhaftung auf einer Seite an Glas, Kunststoff oder proteinhaltiger extrazellulärer Matrix als Zellkulturträger besteht.

Bekannt ist auch ein sogenanntes Sandwichkultursystem, das einen Matrix-Hepatozyt-Matrix-Aufbau besitzt. Dieses System benötigt jedoch zur Realisierung eine Fläche als Unterlage, die zum Auftragen der zweiten oberen Schicht zugänglich sein muß. Vorrichtungen auf der Basis von Hohlfasern (hollow fibers) oder microcarriers ermöglichen zwar theoretisch eine Massenkultur, werden jedoch immer mit herkömmlichen Kulturkonfigurationen verwendet. Dies bedingt einen raschen Funktionsverlust der Hepatozyten, wobei Zusätzlich signifikante Oxygenationsprobleme entstehen. Ein Sandwichkultursystem ist auf diese Weise nicht realisierbar, da es zu Verklebungen kommen würde.

Ein weiterer Nachteil der bekannten Kulturen, insbesondere der Sandwichkulturen, besteht darin, daß die Sauerstoffversorgung der Zellen nur unzureichend gelöst werden konnte. Bereichsweise kam es zu einer Unterversorgung, wohingegen in anderen Bereichen es bei einer Erhöhung der Perfusionsgeschwindigkeit des Kulturmediums zu unerwünschten Erhöhungen von Scherkräften kam.

Um derartige Oxygenationsprobleme von Zellkulturen, insbesondere von Hepatozyten in Kultur, zu umgehen, sind auch bereits gaspermeable Membranen vorgeschlagen worden. Dabei werden die Zellen, die auf der einen Seite der Membrane liegen, entweder durch transmembranären Luftkontakt oder über mit Sauerstoff angereichertem Medium, das an der gegenüberliegenden Seite vorbeiströmt, versorgt. Derartige Einzelmembranen sind jedoch nur für Laborzwecke bzw. nur für geringe Mengen und Größen geeignet.

Ein weiterer Hauptnachteil der bekannten Verfahren und Konstruktionen liegt in den Raumproblemen, d.h. deren großer Platzbedarf. So erfordert z.B. die Beseitigung der Oxygenations- und Nährstoffversorgungsprobleme aufwendige getrennte Pumpenkreisläufe, was zu einer enormen Vergrößerung des Aggregats im Verhältnis zur tatsächlich kultivierten Zellzahl führt. Eine Massenkultur ist auf diese Weise nicht möglich, da ein Gesamtorganersatz, z.B. einer menschlichen Leber, mit einer derartigen Technik den Platzbedarf eines ganzen Hauses besitzen würde.

Aus der EP 0 363 262 ist eine Vorrichtung zur Behandlung von Zellkulturen nach einem Dreikammersystem innerhalb einer Grundeinheit mit einem festen Gehäuse bekannt. In diesem Gehäuse verlaufen zwei voneinander getrennte Einzelmembrane, wodurch drei Kammern entstehen. Diese Membrane sind in den Wänden der Grundeinheit separat verankert. In der mittleren Kammer befindet sich der Zellzuchtraum, während die beiden anderen Kammern Versorgungskammern für die Zellzuchtkammern darstellen.

Nachteilig dabei ist jedoch, daß auch bei dieser Vorrichtung der Raumbedarf erheblich ist. Darüber hinaus ist es mit dieser Vorrichtung nicht möglich, wenigstens annähernd einen "in vivo" Zustand für die Zellen zu erreichen.

Der vorliegenden Erfindung liegt daher die Aufgabe zugrunde, eine Vorrichtung der eingangs erwähnten Art zu schaffen, mit der eine Massenkultur unter sinnvollen räumlichen Verhältnissen und in einem Zustand möglich ist, der soweit wie möglich an einen "in vivo"-Zustand herankommt.

Erfindungsgemäß wird diese Aufgabe dadurch gelöst, daß in das Innere der plattenartigen Zellkulturträger Sauerstoff einleitbar ist, und daß auf dem Zellkulturträger eine die Zellen bedeckende Kollagenschicht aufgetragen ist, wobei direkt oder mit geringem Abstand über der Kollagenschicht der nächste Zellkulturträger angeordnet ist und wobei in die Kollagenschicht oder in den Zwischenraum zwischen der Kollagenschicht und dem nächsten Zellkulturträger Kulturmedium einleitbar ist.

Das erfindungsgemäße Bauprinzip in Form eines Bioreaktors ermöglicht es, auf engstem Raum eine Anordnung zur Behandlung von Zellkulturen zu schaffen, mit dem eine im Vergleich zu den bekannten Lösungen wesentlich höhere Anzahl von Zellen kultiviert werden kann. Durch die erfindungsgemäße Ausgestaltung der Zellkulturträger ist auch eine ausreichende und im wesentlichen überall gleichmäßige Sauerstoffversorgung der Zellkultur geschaffen.

Das erfindungsgemäße Bauprinzip des Bioreaktors imitiert die mikroanatomische und funktionelle Einheit des Leberparenchyms, den Leberlobulus. Dies ermöglicht bei getrennter arterieller und portalvenös-venöser Phase gleichzeitig mehrere wesentliche Vorteile gegenüber bekannten Anlagen und Verfahren. So ist z.B. eine optimale, da unmittelbare, genau dosierbare und gleichmäßig verteilte Versorgung der Hepatozyten mit Sauerstoff möglich. Weiterhin kann die Gesamtzellzahl beliebig hoch den jeweiligen Bedürfnissen angepaßt werden (Massenkultur). Das Totvolumen des Bioreaktors kann minimal gehalten werden.

Wenn in einer sehr vorteilhaften Weiterbildung der Erfindung vorgesehen ist, daß die Zellkultur auf einer ersten Kollagenschicht angeordnet ist, und daß über der Zellkultur eine zweite, obere Kollagenschicht liegt, dann sind die Hepatozyten in einem Kollagensandwich immobilisiert, wodurch eine noch bessere "in vivo"-artige Morphologie und Funktion der Zellen ermöglicht wird.

Eine weitere sehr vorteilhafte Weiterbildung der Erfindung besteht darin, daß aufgrund der erfindungsgemäßen Ausgestaltung des Bioreaktors es auch möglich ist, neben der ersten Zellkultur noch eine zweite Zellkultur zu behandeln, z.B. nichtparenchymale Zellen. Auf diese Weise ist eine Kokultivierung in geordneten dreidimensionalen Strukturen ermöglicht, wie z.B. Sinusoid-Matrix-Hepatozyt-Matrix-nichtparenchymale Zelle-Sinusoid... . Dabei stellt das erste Sinusoid den Sauerstoffzufuhrraum in dem Zellkulturträger dar, während das zweite Sinusoid durch den Spaltraum zwischen der oberen Kollagenschicht und dem nächsten Zellkulturträger bzw. der nächsten Zellkulturschicht gebildet ist.

Wird der erfindungsgemäße Bioreaktor z.B. für die Kultivierung von Hepatozyten verwendet, so stellt der Zellkulturträger einen Querschnitt durch einen Leberlobulus dar. Die Leberzellen (Hepatozyten) sind in konfluierenden Schichten innerhalb der Kollagenmatrix, bestehend aus unterer und oberer Kollagenschicht, analog dem Disseschen Spaltraum verankert. Oberhalb und unterhalb der stapelbaren Zellschichten, befinden sich kapillare Zwischenräume, die den Sinusoiden entsprechen. Diese transportieren portalvenöses Nährmedium als Kulturmedium und, in einer getrennten arteriellen Phase, Sauerstoff durch das Innere der Zellkulturträger herbei. Die Versorgung erfolgt über an der Peripherie des Zellkulturträgers gelegene Portalfelder. Sauerstoff tritt auch in die portalvenöse Phase über und strömt mit dieser in die venöse Phase, die durch den Spaltraum gebildet wird. Analog der Zentralvene wird hier von allen Zellkulturträgern, die die Lobuli imitieren, bzw. Spalträumen das Nährmedium gesammelt und abgeführt.

Das Nährmedium von allen Spalträumen kann dann in einem Reservoir gesammelt und über eine Pumpe z.B. eine peristaltische Pumpe im Kreislauf zum Bioreaktor zurückgeführt werden. Durch eine dazwischen geschaltete Filtereinrichtung können gegebenenfalls Stoffe aus dem Kreislauf ausgeschieden werden. Dies gilt z.B. für Gallenflüssigkeit, sofern man nicht in einfacher Weise das Kulturmedium zwischendurch auswechselt.

Die Zellkulturträger können aus verschiedenen Materialien hergestellt werden. Wesentlich ist lediglich, daß sie bilaminär sind, d.h. daß wenigstens ihre großen, sich gegenüberliegenden Oberflächen gasdurchlässig, jedoch nicht flüssigkeitsdurchlässig sind. Im Bedarfsfalle können die Oberflächen jedoch auch semipermeabel bzw. flüssigkeitsdurchlässig sein. In einem derartigen Falle kann gegebenenfalls ein Stoffaustausch durch die Zellkulturträger erfolgen.

In einer einfachen Ausgestaltung kann dabei vorgesehen sein, daß der Zellkulturträger aus einem oberen und einem unteren Sintermetallband gebildet ist, die durch Abstandshalter voneinander getrennt sind. Zwischen die beiden Bänder wird dann Sauerstoff bzw. Luft mit Kohlendioxyd eingebracht. Je nach Anzahl und Art der Zellkulturträger kann dabei normaler atmospärischer Druck oder ein geringer Überdruck ausreichend sein, damit erreicht wird, daß Sauerstoff durch die gasdurchlässige Schicht diffundiert, und damit in die anliegende Kollagenschicht gelangt.

Derartige Zellkulturträger besitzen eine hohe mechanische Stabilität.

Anstelle von Sintermetall sind als Zellkulturträgermaterial auch Kunststoffe geeignet, die entsprechend gasdurchlässig sind. Hierfür sind z.B. Polypropylen- und Silikonfolien geeignet, welche den zusätzlichen Vorteil besitzen, daß sie lichtdurchlässig sind. Auf diese Weise ist eine lichtmikroskopische Beobachtung der Zellen möglich.

Die erfindungsgemäße Ausgestaltung des Bioreaktors gewährleistet eine weitgehend uniforme Verteilung der Sauerstoffversorgung, da jede Zelle ihren eigenen Sauerstoffplatz hat. Die Sauerstoffversorgung ist zudem über die Zufuhr exakt den Bedürfnissen angepaßt dosierbar und unabhängig von internen strömungstechnischen Verhältnissen.

Durch Übereinanderschichtung einer beliebigen Anzahl Zellkulturträger über eine gemeinsame Sauerstoffzuführung kann die erforderliche Gesamtzellzahl recht einfach beliebig hoch den Bedürfnissen angepaßt werden (Modultechnik). Die das Kulturmedium führenden Spalträume werden über Dichtungsringe von den Zellkulturträgern und deren Sauerstoffzufuhr getrennt.

Die Bedeutung dieser unmittelbaren Oxygenierung mittels bilaminärer Membranen und deren Stapelungsmodus wird klar, wenn man bedenkt, daß so erstmals auch große Zellaggregate in dreidimensionalen Strukturen auf engstem Raum gleichmäßig mit Sauerstoff versorgt werden können. Die Abstände der Platten werden über die Dichtungsringe, z.B. elastische Silikondichtungsringe, die zugleich als Abstandshalter, wie auch als Trennmittel zwischen der Flüssigkeitsphase (venös) und Gasphase (arteriell) wirken, reguliert. Durch Wahl der Größe bzw. der Durchmesser der Dichtungsringe sind die Abstände zwischen den Platten beliebig regulierbar. Im Bedarfsfalle kann nur ein kapillarer Spalt zwischen den Platten verbleiben und es entsteht damit ein "Sinusoid". Ein weiterer Vorteil hierbei ist, daß die Abstände somit unter Einsparung von Totvolumen minimiert werden können.

Statt einer Herstellung der plattenförmigen Zellkulturträger aus Sintermetall können diese gegebenenfalls auch vollständig aus einem nichttoxischem Kunststoff gebildet sein, wobei man vorzugsweise hierfür einen durchsichtigen Kunststoff verwenden wird, da in diesem Falle eine einfache Beobachtung der Kultur möglich ist.

Eine mögliche Ausgestaltung hierfür kann darin bestehen, daß die Zellkulturträger jeweils aus einem Traggerippe gebildet sind, auf bzw. über das jeweils eine gasdurchlässige Membrane gespannt ist.

Das Traggerippe kann dabei aus einem äußeren Ringkörper und einem inneren, die zentrale Bohrung umschließenden Ringkörper bestehen, wobei beide Ringkörper durch speichenförmige Rippen miteinander verbunden sind.

Diese Ausgestaltung ist sehr einfach herstellbar, wobei eine genügend große Stabilität für das Traggerippe gegeben ist, so daß die Zellkulturträger gegebenenfalls mit einer Dicke von weniger als 1 mm hergestellt werden können. Die gasdurchlässige Membrane kann z.B. eine Teflonfolie sein, mit einer Dicke von 0,0025 mm.

In vorteilhafter Weise wird man in wenigstens einen Teil der Rippen die Zufuhröffnungen für Sauerstoff anordnen, wobei in den Rippen Luftleitkanäle angeordnet sind. Die Luftleitkanäle sorgen für die Verteilung des zugeführten Sauerstoffes im Inneren des Zellkulturträgers.

Mit einer erfindungsgemäßen Hepatozytenkultivierung wird bisher z.B. eine bis zu 7 Wochen stabile Funktion erreicht. Zellschichten, die zwischen den Platten liegen, können dabei von zwei Seiten voll ausreichend oxygeniert werden. Dies ermöglicht die dreidimensionale Rekonstruktion einer normalen Leberarchitektur: Sinusoid - Matrix - Hepatozyt - Matrix - nichtparenchymale Zelle - Sinusoid usw. ohne Ischämieprobleme. Über die Zellschichten hinweg entsteht ein freier Gasaustausch mit der arteriellen und portalvenös-venösen Phase.

Alternativ zu der oben angegebenen Reihenfolge kann im Bedarfsfalle auch eine komplette Doppeleinheit jeweils auf der Oberseite eines Zellkulturträgers angeordnet werden. In einem derartigen Falle ist der Schichtaufbau auf der Oberseite eines Zellkulturträgers in folgender Reihenfolge: erste Kollagenschicht - Zellkultur - zweite Kollagenschicht - Zellkultur - dritte Kollagenschicht. Auf die dritte, d.h. die obere Kollagenschicht wird dann der nächste Zellkulturträger aufgesetzt. Dabei ist beim Zusammenbau lediglich darauf zu achten, daß ein Spaltraum zwischen der dritten, oberen Kollagenschicht und der Unterseite des daraufgesetzten Zellkulturträgers erfolgt. In diesen Spaltraum wird dann das Kulturmedium eingebracht. In einem derartigen Falle entfällt somit eine vierte Kollagenschicht, da die mittlere Kollagenschicht beide Zellkulturen nach außen abschließt. Zwar erfolgt die Zufuhr von Kulturmedium daher nicht zentral zwischen den zwei mittleren Kollagenschichten, aber da die Kollagenschichten von dem Kulturmedium problemlos durchsetzt werden können, ist auch in diesem Falle für die untere Zellkultur eine ausreichende Versorgung gegeben.

Die Gas- bzw. Sauerstoffzuführung zu den bilaminären Zellkulturträgern kann an der Peripherie der Zellkultur entsprechend der arteriellen Versorgung im Portalfeld eines Leberlobulus erfolgen. Die Zellkulturträger können sich in einem Glasgefäß als Gehäuse befinden. Kulturmedium kann auch peripher und von unten (portalvenöser Zustrom) zugefügt werden und verteilt sich dann aufsteigend an der Zirkumferenz der Platten und strömt ober- und unterhalb jedes einzelnen bilaminären Zellkulturträgers zu einer zentralen Öffnung. Dort befindet sich z.B. ein sich nach oben verjüngender Kegel. Dies führt damit zu einer nach oben gerichteten Lumenzunahme bzw. Vergrößerung der lichten Weite des Spaltes der "Zentralvene" und ermöglicht bei Wegfall von unnötigem Totvolumen ein geordnetes Abströmen des Kulturmediums vom Umfang zum Zentrum und dann nach oben. Der Bioreaktor entleert sich oben und medial (venöse Phase). Beim Überströmen der Zellschichten vollzieht sich somit der Übergang von der portalvenösen und arteriellen Phase in die venöse Phase. Dies entspricht der "in vivo" Organisation der Leber.

Der erfindungsgemäße Bioreaktor besitzt vielfache Anwendungsmöglichkeiten in der Medizin und Pharmazie.

Eines seiner Schwerpunkte ist dabei der Einsatz als künstliche Leber bzw. als Leberersatz.

Die Metabolisierung einer Vielzahl von Medikamenten erfogt in der Leber. Lipophile Medikamente wie z.B. Ciclosporin oder FK 506 werden speziesabhängig zu verschiedenartigen Metaboliten verstoffwechselt. Diese Metaboliten sind zum Teil für die Wirkung der Substanz aber auch deren Toxizität verantwortlich. Erst durch die klinische Prüfung konnte das andersartige Metabolitenmuster und deren Toxizität beim Menschen im Unterschied zum Tierversuch aufgezeigt werden.

Die Entwicklung eines dynamischen menschlichen Systems, jedoch ohne den Menschen, hätte prinzipielle Vorteile gegenüber dem Tierversuch vor Beginn einer klinischen Prüfung hinsichtlich der Gültigkeit der Ergebnisse. Auch in ethischer Hinsicht stellt sich die Frage, ob wegen der unsicheren Tierversuche, die erstmalige Austestung, insbesonders hoher Wirkstoffkonzentrationen lipophiler Medikamente beim Menschen im Rahmen der klinischen Prüfung noch vertretbar ist.

Weiterhin wird in Großtier- und Primatenversuchen die Entwicklung eines temporären Organersatzes mittels der tierischen Leber angestrebt. Die Kreuzperfusion mit einer Pavianleber wird mit wechselnden Erfolgen schon klinisch verwendet.

Erfindungsgemäß können die pulsatilen Eigenschaften des Bioreaktorkreislaufs ausgenützt und Metaboliten von Pharmaka erfaßt werden. Medikamente und Hormone erreichen "in vivo" pulsatil die Leber.

Diese Bedingungen lassen sich durch den Bioreaktor simulieren. Es können peak- und through-Werte einer Muttersubstanz als auch ihrer Metaboliten ermittelt werden. First pass- und Rezirkulierungsstudien sind möglich. Weiterhin können Metabolitenmuster bei tierischen und menschlichen Zellen ermittelt werden. Eine Untersuchung der direkten Toxizität, insbesonders bei hohen Dosierungen, ist ebenfalls möglich.

Weiterhin ist die kontrollierte Ermittlung einer dosisabhängigen Kinetik der Metabolitenentstehung realisierbar. Eine derartige Situation konnte bisher nur im Tierversuch untersucht werden.

Es bestehen physiologische Unterschiede im Verhalten zwischen humanen und Rattenhepatozyten. Auch der Tierversuch an sich unterliegt diesen Einschränkungen.

Die Anzahl der verwendeten Tiere für pharmäkokinetische und metabolische Untersuchungen in der Industrie ist sehr hoch, da diese fast ausschließlich an Versuchstieren durchgeführt werden. Aus Gründen der Übertragbarkeit auf den Menschen muß eine von 2 Tierarten dem Menschen im Stoffwechselgeschehen ähnlich sein. Dies kann jedoch je nach verwendeter Substanz unterschiedlich sein. Eine geeignete Tierart ist daher nur mit Einschränkungen vorhersagbar. Die Belastung ist selbstredend abhängig von der verwendeten Substanz.

Mit dem erfindungsgemäßen Einsatz des Bioreaktors können nun derartige Versuche gegebenenfalls weitgehend entfallen. So stehen z.B. humane Hepatozyten aus Operationspräparaten nach Leberresektion oder aus nicht oder nur teilweise transplantierten Organen zur Verfügung und können damit in dem erfindungsgemäßen Bioreaktor verwendet werden.

Lebererkrankungen, die zum völligen Ausfall des Organs führen können, sind weit verbreitet und können jeden Menschen akut und unversehens betreffen. Dazu zählen die Hepatitis genauso, wie Lebertumore, als auch Leberschädigungen durch Gifte (Knollenblätterpilz, Alkohol), wie auch Unfälle.

Der künstliche Organersatz durch den erfindungsgemäßen Bioreaktor verfolgt zwei Ziele:
1. Es soll die Wartezeit zwischen dem Eintritt des Leberversagens und der Verfügbarkeit eines neuen Organs bei geplanter Lebertransplantation überbrückt werden. Nicht selten versterben Patienten gerade in dieser Wartefrist.
2. Ein Leberausfall muß nicht immer endgültig sein. Würde man einem Patienten in einer solchen Situation die Möglichkeit einer unterstützenden Behandlung durch einen anderweitigen Organersatz einräumen, könnte der Patient unter Umständen eine derartige Situation überleben. Die eigene Leber hätte Zeit zur Regeneration. Dies ist vor allem denkbar bei akut traumatischen oder toxischen Schädigungen der Leber. Aber auch bei ausgedehnten Tumoroperationen kann die Reserve eines temporären Organersatzes das Operationsrisiko reduzieren oder größere Eingriffe ermöglichen. Der Körper produziert selbst eine große Menge von Wachstumsfaktoren, die ein Nachwachsen der verbliebenen, gesunden Restleber ermöglichen.

Bisherige Behandlungsmethoden beinhalten die Entfernung von Stoffwechselgiften und eine Filtration des Blutes. Leider werden dadurch auch die Wachstumsfaktoren mitentfernt und eine Heilung behindert.

Eine weitere Einsatzmöglichkeit des erfindungsgemäßen Bioreaktors besteht in der großtechnischen Herstellung von Blutgerinnungsfaktoren, wie z.B. Lebersyntheseprodukte. Bisher hat man Blutgerinnungsfaktoren aus dem menschlichen Blut von Blutspendern gewonnen. Dabei besteht jedoch die Gefahr einer Hepatitis-Ansteckung oder einer Aids-Infizierung. Mit dem erfindungsgemäßen Bioreaktor lassen sich nunmehr unabhängig von Blutspendern infektionsfrei z.B. Blutgerinnungsfaktoren gewinnen. Dies ist eine Alternativlösung zu der aufwendigen gentechnischen Gewinnung von Lebersyntheseprodukten.

Nachfolgend ist ein Ausführungsbeispiel der Erfindung anhand der Zeichnung prinzipmäßig dargestellt.

Es zeigt:
- Fig. 1: eine schematische Darstellung des Bauprinzipes des Bioreaktors im Ausschnitt;
- Fig. 2: eine Prinzipdarstellung der Wirkungsweise des Bioreaktors;
- Fig. 3: eine Draufsicht auf eine Zellkulturträgerplatte;
- Fig. 4: den erfindungsgemäßen Bioreaktor in Gesamtansicht;
- Fig. 5: eine Draufsicht auf eine Zellkulturträgerplatte anderer Bauart.

Den Kern des Bioreaktors bilden eine Vielzahl von auf Abstand übereinander angeordneter Zellkulturträger 1, die als silikonbeschichtete Platten aus Sintermetall ausgebildet sind.

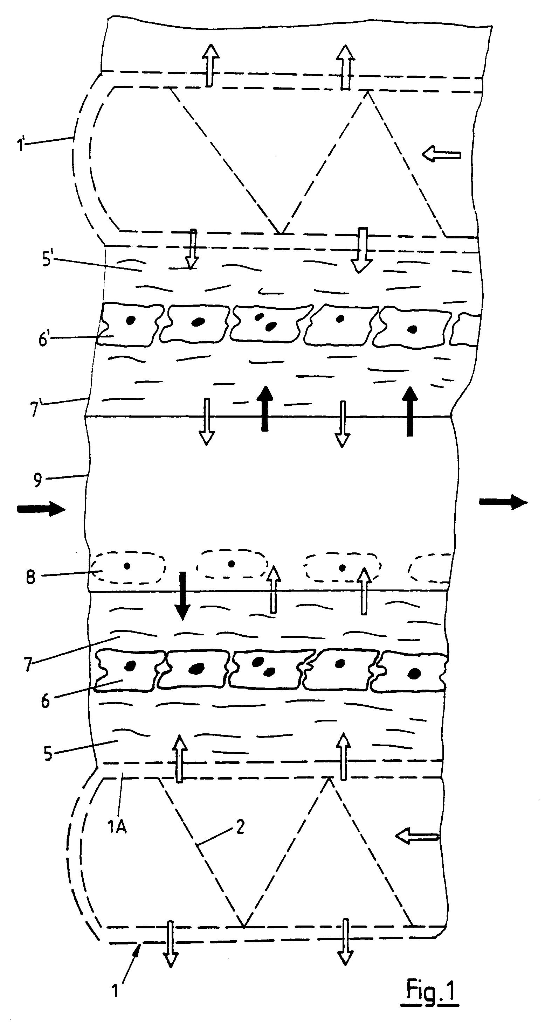

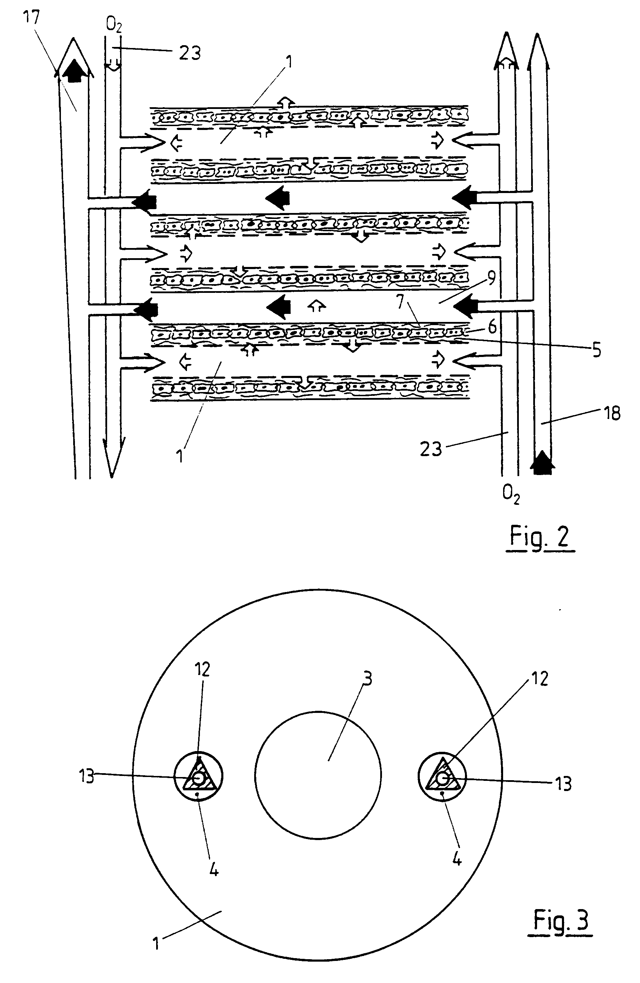

Wie in der vergrößerten Darstellung in Fig. 1 ersichtlich ist, ist jede Platte aus einer dünnen Oberflächenschicht, z.B. einem Sintermetall- oder Kunststoffband 1A gebildet, das an der äußeren Peripherie umgebogen und nach innen zurückgeführt ist. Durch nicht näher dargestellte Abstandshalter 2, die damit einen freien Innenraum zwischen dem oberen Bandteil und dem unteren Bandteil bilden, wird eine Freiraum geschaffen. Wie aus der Fig. 3 ersichtlich ist, ist jede Platte mit einer zentralen Öffnung 3 versehen. Weiterhin sind zwei diametral sich gegenüberliegende Bohrungen 4 vorhanden, wobei sich die Bohrungen 4 zwischen dem äußeren Umfang und der zentralen Öffnung 3 befinden. Die Bohrungen 4 dienen zur Zufuhr von Sauerstoff in das Innere der Platten 1. Aus diesem Grunde sollten die Bohrungen 4 in einer derartigen Verteilung in jeder Platte 1 angeordnet sein, daß eine ausreichende und gleichmäßige Sauerstoffverteilung erfolgen kann.

Auf die Oberseite des oberen Bandes 1A wird eine erste hydrierte Kollagenschicht 5 (z.B. Protein von Haut, Knochen und Knorpel) in einer Stärke von ca. 0,5 mm aufgetragen. Auf diese Kollagenschicht 5 folgt eine Zellkulturschicht 6, z.B. Hepatozyten. Auf die Zellkulturschicht 6 ist eine zweite, obere Kollagenschicht 7 aufgebracht. Gegebenenfalls kann darüber nochmals eine Schicht aus z.B. nichtparenchymalen Zellen 8 aufgebracht werden (siehe gestrichelte Darstellung in der Fig. 1). Die beiden Kollagenschichten 5 und 7 stellen für die Gas- bzw. Luftzufuhr und die Nährstoffdiffusion zu den Hepatozyten keine wesentliche Barriere dar. Selbst große Moleküle können problemlos durch diese Schicht hindurchtreten. Da die Zellen 6 dem Zellkulturträger 1 als Sauerstoffträger mittels der ersten Kollagenschicht 5 direkt aufliegen, ergibt sich dadurch hinsichtlich der Gasdiffusion eine Situation wie in einem Inkubator nach Entfernung des Mediums. Dies ist dort die Voraussetzung für die Optimierung einer Sauerstoffversorgung bei der Kultivierung großer Zellzahlen in konfluierenden Schichten. In dem Bioreaktor wird jedoch nicht auf das nährstofftragende Kulturmedium verzichtet. Dieses wird über den geringen Abstand bzw. Spalt zwischen der oberen Kollagenschicht 7 und einer weiteren Auflageeinheit zugeführt. Die weitere (und folgende) Auflageeinheit besteht wiederum aus einer unteren bzw. inneren Kollagenschicht 5', die auf dem mit Abstand über dem ersten Zellkulturträger 1 angebrachten weiteren Zellkulturträger 1' aufgebracht ist. Auf die innere Kollagenschicht 5' folgt wiederum die Zellkulturschicht 6', die außenseitig durch die zweite bzw. äußere Kollagenschicht 7' abgedeckt ist. Diese Einheit ist praktisch über Kopf angeordnet und die Versorgung mit z.B. Nährstoff oder Plasma erfolgt über den gemeinsamen Spaltraum 9.

In einer Abwandlung des in der Fig. 1 dargestellten Aufbaues kann auch die dargestellte Doppeleinheit mit zwei Schichten aus Zellkulturen 6 und 6', die jeweils zwischen zwei Zellkulturträger 1 bzw. 1' angeordnet ist, von der Oberseite des unteren Zellkulturträgers 1 vollständig aufgebaut sein. Dieses Verfahren bietet sich insbesondere dann an, wenn die Gefahr besteht, daß die Kollagenschicht 5' nicht oder nur schlecht auf der Unterseite des jeweils darüber angeordneten Zellkulturträgers 1' haftet. Außerdem wird das Arbeiten am bzw. der Aufbau des Bioreaktors damit gegebenenfalls erleichtert.

In diesem Falle wird statt dem Spaltraum 9 auf die obere Kollagenschicht 7 direkt die obere Zellkultur 6' aufgelegt, wie es z.B. durch die nichtparenchymale Zellkulturschicht 8 dargestellt ist. Auf die obere Zellkulturschicht 6' wird dann die obere Kollagenschicht 5' aufgebracht und anschließend der nächste Zellkulturträger 1' aufgesetzt. Dabei ist lediglich darauf zu achten, daß beim Zusammenbau zwischen der oberen Kollagenschicht 5' und der Unterseite des Zellkulturträgers 1' ein geringer Zwischenraum zur Zufuhr von Kulturmedium verbleibt. In diesem Falle erfolgt somit die Nährstoffversorgung der Zellkulturschichten 6 und 6' von oben her durch die Kollagenschicht 5'. Die Kollagenschicht 7' entfällt und es gibt nun eine mittlere Kollagenschicht 7.

Wie ersichtlich, wird der Sauerstoffbedarf stoffwechselaktiven Hepatozyten unabhängig vom Medium und der Durchflußgeschwindigkeit des Mediums garantiert. Die Zellkulturträger 1 selbst stellen den Oxygenator dar, wodurch auch konfluierende Zellschichten 6 unter Einsatz des Kapillarnetzes exakt dosiert mit Sauerstoff versorgt werden können.

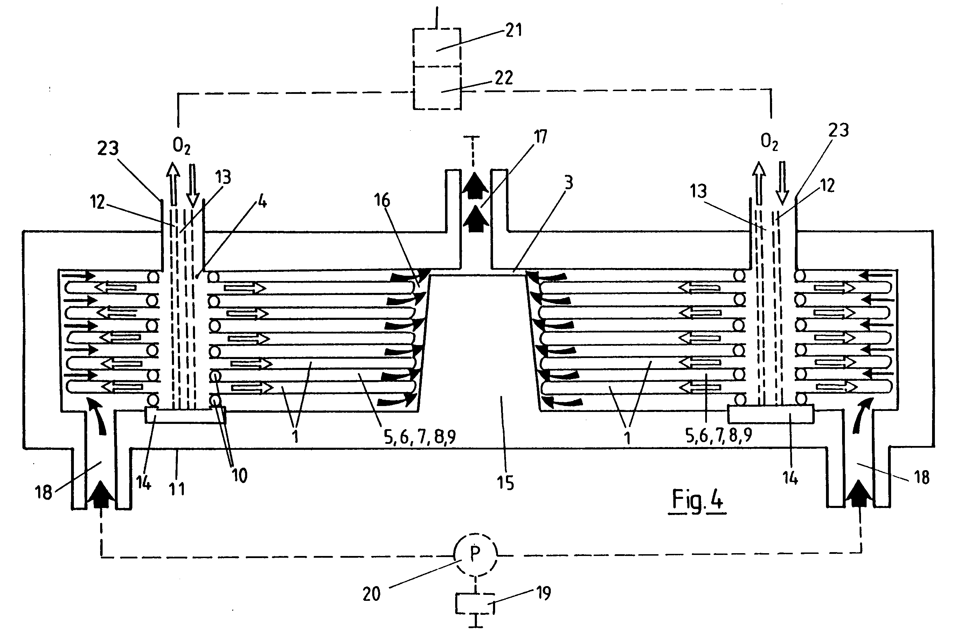

In der Fig. 2 ist schematisch der Aufbau des Bioreaktors dargestellt. Dabei stellen die schwarzen Pfeile den Weg bzw. die Fließrichtung des Kulturmediums dar, während die weißen Pfeile den Verlauf des Sauerstoffes darstellen. Gleiches gilt auch für die übrigen Pfeildarstellungen in den Fig. 1 und 4.

Dabei wird Sauerstoff über eine gemeinsame Sauerstoffleitung 23, die im Zusammenhang mit der Fig. 4 näher erläutert wird, eingebracht. Kulturmedium wird ebenfalls über eine gemeinsame Leitung 18 in gleichmäßiger Verteilung allen Spalträumen 9 zugeführt.

Der Gesamtaufbau des Bioreaktors ist aus der Fig. 4 ersichtlich. Wie dargestellt, wird eine Vielzahl von Zellkulturträger 1 auf Abstand übereinander angeordnet, wobei Abdichtringe in Form von Silikondichtungsringe 10, die um die Sauerstoffbohrungen 4 jeder Platte gelegt werden, sowohl für eine Einhaltung eines Abstandes zwischen den Platten 1 als auch für eine Abdichtung zwischen der Flüssigphase (venös) und der Gasphase (arteriell) sorgen. Die Wahl der Größe bzw. der Dicke der Silikondichtungsringe 10, die beim Zusammenbau der Platten verpreßt werden, sorgt auch dafür, daß ein kapillarer Spaltraum 9 zwischen den Platten verbleibt und damit das "Sinusoid" bildet.

Die Zufuhr von Sauerstoff in das Gehäuse 11 des Bioreaktors erfolgt über eine Sauerstoffleitung 23 in die fluchtend übereinanderliegenden Sauerstoffbohrungen 4 der Zellkulturträger 1. Zur Verbindung der Zellkulturträger 1 miteinander dient ein Stab 12, der - im Querschnitt gesehen - dreiecksförmig ist. Auf diese Weise verbleibt zwischen den Bohrungswandungen der Sauerstoffbohrungen 4 und dem Stab 12 genügend Freiraum zum Einleiten von Sauerstoff (siehe Fig. 3). Der Stab 12 ist mit einer zentralen Bohrung 13 versehen, durch die eine Spannschraube gesteckt wird, welche in nicht mehr dargestellter Weise zusammen mit einer Kontermutter bzw. Konterplatte 14 auf der Unterseite für eine Verbindung und eine gasdichte Verspannung der Silikondichtungsringe 10 sorgt. In den Freiraum, der durch die fluchtend übereinanderliegenden zentralen Öffnungen 3 der Zellkulturträger 1 entsteht, ist ein Kegel z.B. ein Glaskegel 15 eingesetzt. Der Glaskegel 15 besitzt einen geringeren Durchmesser wie die Durchmesser der zentralen Öffnungen 3, wodurch ein Spaltraum 16 entsteht. Da sich der Kegel 15 nach oben verjüngt, vergrößert sich auf diese Weise der Spaltraum 16, wodurch für einen guten und gleichmäßigen Ablauf des Kulturmediums über eine gemeinsame Ablaufleitung 17 gesorgt ist.

Wie ersichtlich, strömt das Kulturmedium an beiden Seiten des Bioreaktors über Zulaufleitungen 18 in das Gehäuse 11 ein und von dort aus von außen her peripher längs des Spaltraumes 9 nach innen, wo es über den Spaltraum 16 abfließt.

Das Kulturmedium kann im Kreislauf geführt werden, wobei ein Sammelreservoir 19 und eine nachfolgende Pumpe 20 vorgesehen ist, die über die gestrichelt dargestellte Leitung das Kulturmedium wieder zu den Zulaufleitungen 18 führt. Gegebenenfalls kann in der Kreislaufleitung auch noch eine Filtereinrichtung zum Abscheiden von unerwünschten Bestandteilen aus dem vom Bioreaktor auftretenden Kulturmedium angeordnet sein.

Sofern der Bioreaktor nicht in einem Inkubator angeordnet ist, wird man im allgemeinen den Sauerstoff, der mit ca. 5 % Kohlendioxyd versetzt sein kann, über einen Vorwärmer 21 und einem Befeuchter 22 dem Bioreaktor zuführen. Die den Bioreaktor verlassende Luft kann durch eine Wassersäule hindurchgeführt werden. Auf diese Weise wird ein Kohlendioxydaustausch mit der umgebenden Luft verhindert, wodurch der gewünschte hohe Kohlendioxydgehalt praktisch in einer Art geschlossenem System stabilisiert werden kann.

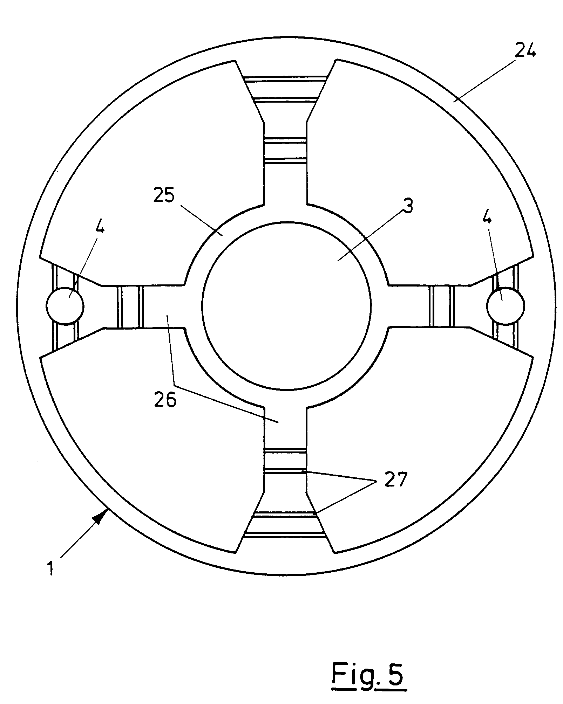

In der Fig. 5 ist eine konstruktive Ausgestaltung eines scheibenförmigen Zellkulturträgers 1 dargestellt, der vorzugsweise aus einem nichttoxischen Kunststoff besteht. Als Traggerippe dienen dabei ein äußerer Ringkörper 24 und ein innerer Ringkörper 25, der die zentrale Bohrung 3 umschließt. Die Verbindung der beiden Ringkörper 24 und 25 miteinander erfolgt durch speichenförmige Rippen 26. In zwei gegenüberliegenden Rippen 26 sind die Zufuhröffnungen 4 für Sauerstoff angeordnet. Dieses Traggerippe wird auf der Oberseite und auf der Unterseite durch eine transparente Membrane, z.B. einer Teflonfolie, überspannt. Damit der Sauerstoff im Inneren des Zellkulturträgers 1 gleichmäßig verteilt wird, sind die Rippen 26 jeweils mit einem oder mehreren Luftleitkanälen 27 auf ihren Oberflächen (Ober- und Unterseite) versehen, welche die Rippen in ihrer Breite jeweils durchsetzen.

Die Dicken bzw. Abstände der einzelnen Schichten sind in Abhängigkeit von den zu behandelnden Zellkulturen zu wählen. Für die Kultivierung von Hepatozyten haben sich Dicken von ca. 0,5 mm für die Zellkulturträger 1 als sehr geeignet herausgestellt, wobei die gasdurchlässige Oberflächenschicht bzw. das Sinterband gegebenenfalls nur 0,1 mm oder weniger betragen kann. Für die beiden Kollagenschichten haben sich Dicken von jeweils 0,4 bis 0,6 mm, vorzugsweise 0,5 mm als geeignet herausgestellt. Die Zellkulturschicht kann eine Dicke von 0,002 bis 0,003 mm besitzen. Für den Spaltraum 9 genügen ebenfalls wenige zehntel Millimeter.

Schätzt man den Gehalt an Hepatozyten von 1 g Leber auf 100 Millionen, so würde die Leber einer 250 g schweren Ratte bei 8 bis 10 g 800 Millionen bis 1 Milliarde Hepatozyten enthalten. Versuche haben gezeigt, daß dies der Kapazität eines Bioreaktors mit einer Innenhöhe von ca. 30 mm und einem Durchmesser der plattenförmigen Zellkulturträger von ca. 10 bis 15 cm hierfür ausreicht.

Um die Aufgaben einer menschlichen Leber übernehmen zu können, bzw. als künstliche menschliche Leber, wären schätzungsweise ca. 2.000 übereinanderliegende Platten erforderlich, was bei einer Anordnung der Platten in 4 Säulen, z.B in einer Kleeblattstruktur, eine Gesamthöhe von ca. 80 bis 100 cm ergeben würde. Der Eigenvolumenbedarf des Bioreaktors an Kulturmedium wäre dabei mit ca. 10 l relativ gering. Über ein Reservoir könnte dabei der Stoffaustausch mit dem Patientenplasma erfolgen.

## Patentansprüche

1. Vorrichtung zur Massenkultur von Zellen, insbesondere von Hepatozyten, auf Zellkulturträgern, wobei wenigstens ein Teil der Oberflächen der Zellkulturträger gasdurchlässig ist,
**dadurch gekennzeichnet,** daß in das Innere der plattenartigen Zellkulturträger (1) Sauerstoff einleitbar ist und daß auf dem Zellkulturträger (1) eine die Zellen bedeckende Kollagenschicht (5,7) aufgetragen ist, wobei direkt oder mit geringem Abstand über der Kollagenschicht (5,7) der nächste Zellkulturträger (1) angeordnet ist und wobei in die Kollagenschicht (5,7) oder in den Zwischenraum zwischen der Kollagenschicht (5,7) und dem nächsten Zellkulturträger (1) Kulturmedium einleitbar ist.

2. Vorrichtung nach Anspruch 1,
**dadurch gekennzeichnet**, daß die Zellkultur (6) auf einer ersten Kollagenschicht (5) angeordnet ist, und daß über der Zellkultur (6) eine zweite, obere Kollagenschicht (7) liegt.

3. Vorrichtung nach Anspruch 1 oder 2,
**dadurch gekennzeichnet**, daß zwischen zwei Kollagenschichten (7,7') oder zwischen einer Kollagenschicht (5') und der Unterseite eines Zellkulturträgers (1') ein Zwischenraum (9) zur Zufuhr von Kulturmedium angeordnet ist.

4. Vorrichtung nach Anspruch 1, 2 oder 3,
**dadurch gekennzeichnet**, daß auf der ersten Zellkultur (6) eine zweite Zellkultur (8) angeordnet ist.

5. Vorrichtung nach Anspruch 4,
**dadurch gekennzeichnet**, daß die zweite Zellkultur (8) aus nichtparenchymalen Zellen gebildet ist.

6. Vorrichtung nach einem der Ansprüche 1 bis 5,
**dadurch gekennzeichnet**, daß die Zellkulturträger (1) aus Sintermetall gebildet sind.

7. Vorrichtung nach Anspruch 1,
**dadurch gekennzeichnet**, daß jeder Zellkulturträger (1) aus einer oberen und einer unteren gasdurchlässigen Schicht oder Band (1A) gebildet ist, die oder das durch Abstandshalter voneinander getrennt sind.

8. Vorrichtung nach Anspruch 7,
**dadurch gekennzeichnet**, daß die Schicht (1A) aus Sintermetall gebildet ist.

9. Vorrichtung nach Anspruch 7,
**dadurch gekennzeichnet**, daß die Zellkulturträger (1) aus einem gasdurchlässigen Kunststoff gebildet sind oder einen gasdurchlässigen Kunststoff aufweisen.

10. Vorrichtung nach Anspruch 9,
**dadurch gekennzeichnet**, daß die Zellkulturträger (1) aus einem Traggerippe (24,25,26) gebildet sind, auf oder über das jeweils eine gasdurchlässige Membrane gespannt ist.

11. Vorrichtung nach Anspruch 10,
**dadurch gekennzeichnet**, daß das Traggerippe aus einem äußeren Ringkörper (24) und einem inneren, die zentrale Bohrung (3) umschließenden Ringkörper (25) besteht, wobei beide Ringkörper (24,25) durch speichenförmige Rippen (26) miteinander verbunden sind.

12. Vorrichtung nach Anspruch 11,
**dadurch gekennzeichnet**, daß in wenigstens einem Teil der Rippen (26) die Zufuhröffnungen (4) für Sauerstoff angeordnet sind, und daß in den Rippen (26) Luftleitkanäle (27) angeordnet sind.

13. Vorrichtung nach einem der Ansprüche 9 bis 12,
**dadurch gekennzeichnet**, daß die gasdurchlässigen Oberflächen durch eine Silikon- oder Polypropylenfolie gebildet sind.

14. Vorrichtung nach einem der Ansprüche 1 bis 13,
**dadurch gekennzeichnet**, daß zwischen den einzelnen Zellkulturträgern (1) als Abstandshalter und/oder zur Abdichtung dienende Abdichtringe (10) angeordnet sind.

15. Vorrichtung nach einem der Ansprüche 1 bis 14,
**dadurch gekennzeichnet**, daß sie bausteinartig mit einer Vielzahl von in einem Gehäuse übereinander angeordneten Zellkulturträgern (1) ausgebildet ist, mit wenigstens einer Zufuhrleitung (23) für Sauerstoff und mit wenigstens einer Zulaufleitung (18) und wenigstens einer Ablaufleitung (17) für Kulturmedium.

16. Vorrichtung nach Anspruch 15,
**dadurch gekennzeichnet**, daß die Zellkulturträger (1) wenigstens annähernd eine Scheibenform aufweisen, wobei die Zufuhr von Kulturmedium umfangsseitig und der Ablauf vom zentralen Bereich der an dieser Stelle mit einem Freiraum versehenen Zellkulturträger (1) erfolgt und daß die Zellkulturträger (1) jeweils mit wenigstens zwei gleichmäßig über den Umfang der Zellkulturträger (1) angeordnete Zufuhröffnungen (4) für Sauerstoff versehen sind.

17. Vorrichtung nach Anspruch 15 oder 16,
**dadurch gekennzeichnet**, daß die Zellkulturträger (1) jeweils mit einer zentralen Bohrung (3) versehen sind, und daß sich durch die zentralen Bohrungen ein sich in Ablaufrichtung verjüngender Kegel (15) angeordnet ist, der einen derartigen Durchmesser aufweist, daß ein Ringraum zu den Wänden der zentralen Bohrungen (3) der Zellkulturträger (1) verbleibt.

18. Vorrichtung nach einem der Ansprüche 15 bis 17,
**dadurch gekennzeichnet**, daß die Zellkulturträger (1) durch eine sich durch die Sauerstoffzufuhröffnungen (4) erstreckende Spanneinrichtung (12,13) miteinander zu einer Einheit verbunden sind.

19. Vorrichtung nach Anspruch 18,
**dadurch gekennzeichnet**, daß die Sauerstoffzufuhröffnungen (4) kreisförmig ausgebildet sind und die Spanneinrichtung einen von der Kreisform abweichenden Führungsstab (12) aufweist, durch dessen Inneres eine Spannschraube gesteckt ist.

20. Vorrichtung nach einem der Ansprüche 15 bis 19,
**dadurch gekennzeichnet**, daß mehrere aus Zellkulturträgern (1) gebildete Türme kleeblattartig nebeneinander angeordnet sind.

21. Vorrichtung nach einem der Ansprüche 1 bis 20,
**dadurch gekennzeichnet**, daß das Nährmedium über ein Sammelreservoir (19) und eine Pumpe (20) im Kreislauf geführt ist.

22. Vorrichtung nach Anspruch 21,
**dadurch gekennzeichnet**, daß in dem Kreislauf eine Filtereinrichtung für abzuscheidende Stoffe angeordnet ist.

## Claims

1. A device for the mass culture of cells, especially hepatocytes, on cell culture slides, at least a part of the surface of the cell culture slides being gas-permeable,
characterised in that oxygen can be introduced inside the plate-like cell culture slides (1), and in that a collagen layer (5,7) covering the cells is applied to the cell culture slide (1), the next cell culture slide (1) being disposed directly or a slight distance above the collagen layer (5,7) and it being possible to introduce culture medium into the collagen layer (5,7) or into the space between the collagen layer (5,7) and the next cell culture slide (1).

2. A device according to claim 1,
characterised in that the cell culture (6) is disposed on a first collagen layer (5), and in that a second, upper collagen layer (7) lies over the cell culture (6).

3. A device according to claim 1 or 2,
characterised in that a space (9) for the supply of culture medium is provided between two collagen layers (7,7') or between a collagen layer (5') and the under side of a cell culture slide (1').

4. A device according to claim 1, 2 or 3,
characterised in that a second cell culture (8) is disposed on the first cell culture (6).

5. A device according to claim 4,
characterised in that the second cell culture (8) is formed from non-parenchymal cells.

6. A device according to one of claims 1 to 5,
characterised in that the cell culture slides (1) are formed from sintered metal.

7. A device according to claim 1,
characterised in that each cell culture slide (1) is formed from an upper and a lower gas-permeable layer or strip (1A), which are separated from one another by spacers.

8. A device according to claim 7,
characterised in that the layer (1A) is formed from sintered metal.

9. A device according to claim 7,
characterised in that the cell culture slides (1) are formed from a gas-permeable plastic or comprise a gas-permeable plastic.

10. A device according to claim 9,
characterised in that the cell culture slides (1) are formed from a supporting frame (24,25,26), on or over which a gas-permeable membrane is stretched.

11. A device according to claim 10,
characterised in that the supporting frame consists of an outer annular member (24) and an inner annular member (25) surrounding the central bore (3), both annular members (24,25) being connected to one another by spoke-shaped ribs (26).

12. A device according to claim 11,
characterised in that the supply appertures (4) for oxygen are disposed in at least a part of the ribs (26), and in that air ducts (27) are provided in the ribs (26).

13. A device according to one of claims 9 to 12,
characterised in that the gas-permeable surfaces are formed by a silicon or polypropylene film.

14. A device according to one of claims 1 to 13,
characterised in that sealing rings (10) used as spacers and/or for sealing are disposed between the individual cell culture slides (1).

15. A device according to one of claims 1 to 14,
characterised in that it is constructed in modular fashion with a plurality of cell culture slides (1) disposed above one another in a housing, having at least one supply line (23) for oxygen and having at least one inlet line (18) and at least one outlet line (17) for culture medium.

16. A device according to claim 15,
characterised in that the cell culture slides (1) have at least roughly a disc shape, the supply of culture medium occurring on the peripheral side and the discharge occurring from the central region of the cell culture slides (1) provided with a free space at this place, and in that each cell culture slide (1) is provided with at least two supply apertures (4) for oxygen disposed uniformly over the circumference of the cell culture slides (1).

17. A device according to claim 15 or 16,
characterised in that each cell culture slide (1) is provided with a central bore (3), and in that a cone (15) tapering in the discharge direction, which has such a diameter that an annular space to the walls of the central bores (3) of the cell culture slides (1) remains, is disposed through the central bores.

18. A device according to one of claims 15 to 17,
characterised in that the cell culture slides (1) are connected to one another to form a unit by a tensioning device (12,13) extending through the oxygen supply apertures (4).

19. A device according to claim 18,
characterised in that the oxygen supply apertures (4) have a circular construction and the tensioning device has a guide rod (12) deviating from the circular shape, through the interior of which a straining screw is placed.

20. A device according to one of claims 15 to 19,
characterised in that several towers formed from cell culture slides (1) are disposed in cloverleaf fashion next to one another.

21. A device according to one of claims 1 to 20,
characterised in that the nutrient medium is circulated via a collecting basin (19) and a pump (20).

22. A device according to claim 21,
characterised in that a filter device is provided in the circulation for substances to be collected.

## Revendications

1. Dispositif pour la culture massive de cellules, en particulier d'hépatozytes sur des supports de culture, dans lequel au moins une partie des surfaces des supports de culture est perméable aux gaz,
**caractérisé en ce que** de l'oxygène peut être amené à l'intérieur des supports de culture (1) en forme de plaques, en ce qu'une couche de collagène (5, 7) est déposée sur les supports de culture (1) de manière à recouvrir les cellules, le support de culture (1) adjacent étant disposé directement sur ou à faible distance de la couche de collagène (5, 7), et en ce que de la matière nutritive est amenée dans la couche de collagène (5, 7) ou dans l'espace séparant la couche de collagène (5, 7) du support de culture (1) adjacent.

2. Dispositif selon la revendication 1,
**caractérisé en ce que** la culture cellulaire (6) est disposée sur une première couche de collagène (5) et en ce qu'une deuxième couche supérieure de collagène (7) est déposée sur la culture cellulaire (6).

3. Dispositif selon la revendication 1 ou 2,
**caractérisé en ce qu'**entre deux couches de collagène (7, 7') ou entre une couche de collagène (5') et la face inférieure d'un support de culture (1') un espace intermédiaire (9) est ménagé pour assurer l'amenée de matière nutritive.

4. Dispositif selon l'une des revendications 1, 2 ou 3,
**caractérisé en ce qu'**une deuxième culture cellulaire (8) est disposée sur la première culture cellulaire (6).

5. Dispositif selon la revendication 4,
**caractérisé en ce que** la deuxième culture cellulaire (8) est constituée de cellules non parenchymales.

6. Dispositif selon l'une des revendications 1 à 5,
**caractérisé en ce que** le support de culture (1) est constitué par du métal fritté.

7. Dispositif selon la revendication 1,
**caractérisé en ce que** chaque support de culture (1) est constitué par une couche ou une bande (1A) supérieure perméable aux gaz et par une couche inférieure, qui sont séparées par des distanceurs.

8. Dispositif selon la revendication 7,
**caractérisé en ce que** la couche (1A) est constituée par du métal fritté.

9. Dispositif selon la revendication 7,
**caractérisé en ce que** les supports de culture (1) sont constitués par un matériau synthétique perméable aux gaz ou comportent un matériau synthétique perméable aux gaz.

10. Dispositif selon la revendication 9,
**caractérisé en ce que** les supports de culture (1) sont constitués par des nervures de support (24, 25, 26) sur lesquelles ou par-dessus lesquelles est tendue une membrane perméable aux gaz.

11. Dispositif selon la revendication 10,
**caractérisé en ce que** les nervures de support comportent un corps annulaire extérieur (24) et un corps annulaire intérieur (25) entourant un évidement central (3), les deux corps annulaires (24, 25) étant reliés par des nervures (26) en forme de rayons.

12. Dispositif selon la revendication 11,
**caractérisé en ce qu'**au moins une partie des nervures (26) comporte des ouvertures d'amenée (4) d'oxygène, et en ce que les nervures (26) comportent des canaux d'amenée d'air (27).

13. Dispositif selon l'une des revendications 9 à 12,
**caractérisé en ce que** les surfaces perméables aux gaz sont constituées d'une feuille de silicone ou de polypropylène.

14. Dispositif selon l'une des revendications 1 à 13,
**caractérisé en ce que** des joints d'étanchéité (10) sont disposés à titre de distanceurs et/ou pour assurer l'étanchéité entre les différents supports de culture (1).

15. Dispositif selon la revendication 1 à 14,
**caractérisé en ce qu'**il comporte une pluralité de supports de culture (1) identiques disposés dans un boîtier pourvu d'au moins un conduit d'amenée (23) pour l'oxygène et d'au moins un conduit d'amenée (18) et d'au moins un conduit de retour (17) pour la matière nutritive.

16. Dispositif selon la revendication 15,
**caractérisé en ce que** les supports de culture (1) ont au moins approximativement la forme d'un disque, l'amenée de la matière nutritive s'effectuant à la périphérie et l'évacuation de cette matière dans la zone centrale, les supports de culture (1) présentant un vide dans cette zone, et en ce que ces supports de culture (1) sont respectivement équipés d'au moins deux conduits d'amenée (4) pour l'oxygène, ces conduits d'amenée s'étendant régulièrement sur toute la périphérie des supports de culture (1).

17. Dispositif selon les revendications 15 ou 16,
**caractérisé en ce que** les supports de culture (1) sont pourvus chacun d'un évidement central (3), et en ce qu'un cône (15) se rétrécissant vers le bas est défini par lesdits évidements centraux, ce cône ayant un diamètre tel qu'il subsiste un espace annulaire entre les parois des évidements centraux (3) des supports de culture (1).

18. Dispositif selon l'une des revendications 15 à 17,
**caractérisé en ce que** les supports de culture (1) sont couplés entre eux pour former une unité par l'intermédiaire d'un dispositif de tension (12, 13) s'étendant à travers les ouvertures d'amenée d'oxygène (4).

19. Dispositif selon la revendication 18,
**caractérisé en ce que** les ouvertures d'amenée d'oxygène (4) ont une forme circulaire et en ce que le dispositif de tension comporte une tige de guidage (12) présentant une forme non circulaire et s'étendant à travers l'intérieur d'une vis de tension.

20. Dispositif selon l'une des revendications 15 à 19,
**caractérisé en ce que** plusieurs tours formées de supports de culture (1) sont disposées côte à côte en feuille de trèfle.

21. Dispositif selon l'une des revendications 1 à 20,
**caractérisé en ce que** la matière nutritive est mise en circulation à travers un réservoir collecteur (19) et au moyen d'une pompe (20).

22. Dispositif selon la revendication 21,
**caractérisé en ce que** le circuit de la matière nutritive comporte une installation de filtration pour séparer des matières à éliminer.
